# EUROPEAN PATENT APPLICATION

(11) **EP 1 514 567 A1**
(43) Date of publication of application: **16.03.2005**
(21) Application number: 04020531.2
(22) Date of filing: 30.08.2004
(51) Int. Cl.: A61M 5/42

(54) **Device for attenuating pain of injections**

(30) Priority: 04.09.2003 IT MI20031706
(71) Applicant: Romano', Carlo Luca, Dott., 20121 Milano (IT)
(72) Inventor: Romano', Carlo Luca, Dott., 20121 Milano (IT)
(74) Representative: Cicogna, Franco

(57) **Abstract**

An auxiliary device (1) for attenuating pain of injections, characterized in that said auxiliary device comprise a plate portion (2) including a plurality of pointed elements (3) adapted to be pressed on a user skin at a region about or near an injection point, to reduce a needle puncture pain; said device can be used for attenuating pain of injections, traumas, small lesions, hair removing operations, and inflammation conditions.

## Description

### BACKGROUND OF THE INVENTION

The present invention relates to an auxiliary device for attenuating pain of injections, traumas, small lesions, hair removing operations, and inflammation conditions.

As is known, a lot of persons don't bear injections, because they are afraid of the pain which could be caused by the penetration of the needle.

This problem, in particular, greatly affects some patients, such as children. Thus, it would be very advantageous to provide a device adapted to reduce or eliminate this anxiety status in such patients.

### SUMMARY OF THE INVENTION

Accordingly, the aim of the present invention is to design an auxiliary device for performing injections, adapted to reduce pain associated with injections by needles of any types, shape and/or use, both in a human and veterinary field.

Within the scope of the above mentioned aim, a main object of the present invention is to provide such a device which is construction-wise very simple and inexpensive.

Another object of the present invention is to provide such a device which can be used in a very easy manner, even by unskilled persons or users.

According to one aspect of the present invention, the above mentioned aim and objects, as well as yet other objects, which will become more apparent hereinafter, are achieved by an auxiliary device for performing injections, characterized in that said device comprise a plate portion including a plurality of pointed elements, which can be pressed on a user skin about an injection point.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:

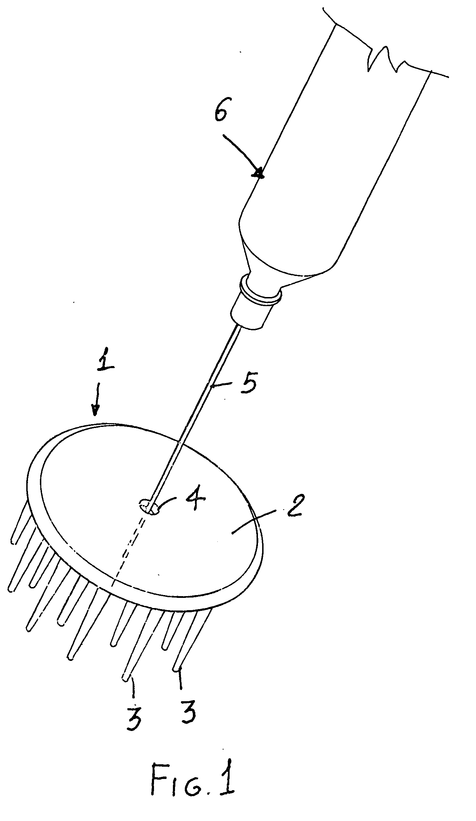

Figure 1 is a perspective view illustrating the auxiliary device according to the present invention and an injection syringe;

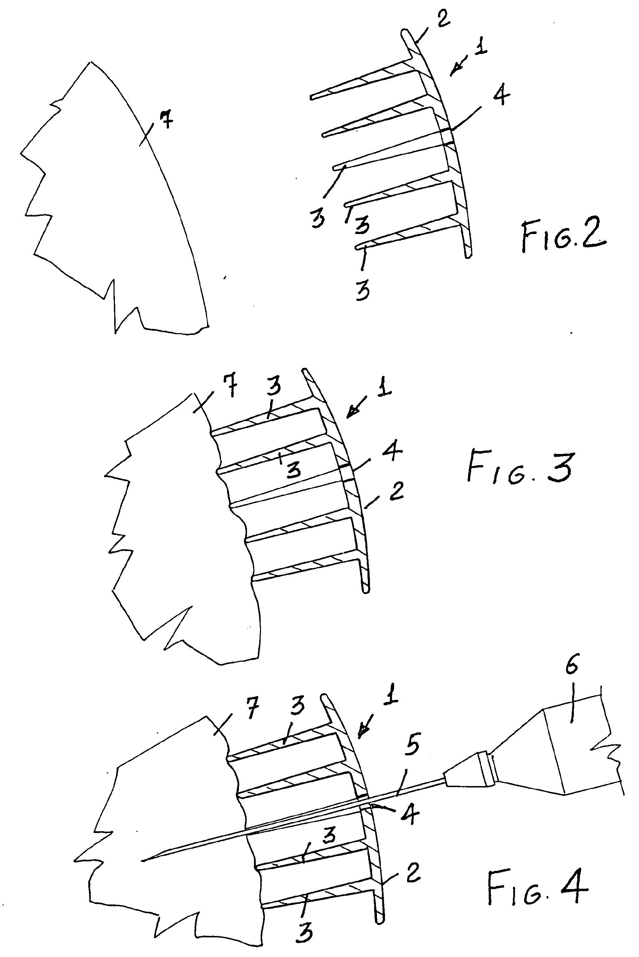

Figure 2 is a cross-sectional view illustrating the device and a skin portion thereon the auxiliary device will be applied;
Figure 3 is a view similar to figure 2, illustrating the application of the device on the skin;
Figure 4 is a view similar to figure 3, illustrating an injection step by the auxiliary device according to the present invention;
Figure 5 shows a modified embodiment, in perspective, of the device according to the present invention, in which the needle housing and locating seat is formed in a notch or incision having a V shape, formed at the plate-like portion 2;
Figures 6, 7 and 8 show, by cross-sectioned side views, the device according to the present invention, in the embodiment thereof shown in figure 5.

### DESCRIPTION OF THE PREFERRED EMBODIMENT

With reference to the number references of the above mentioned figures, the auxiliary device, according to the invention, which has been generally indicated by the reference number 1, comprises a plate-like portion 2, provided for supporting a plurality of pointed elements 3.

The auxiliary device can moreover comprise a hole 4, for allowing a needle 5 of a syringe 6 to be introduced therethrough, to perform an injection, as it will be disclosed in a more detailed manner thereinafter.

Advantageously, the plate 2 and the pointed elements 3 be made as a single body starting from several materials, for example plastics materials.

In a basic embodiment, shown in the drawing figures, the device comprises a disc 2, having a plurality of points 3, made of a plastics material and molded, for example, by an injection molding method.

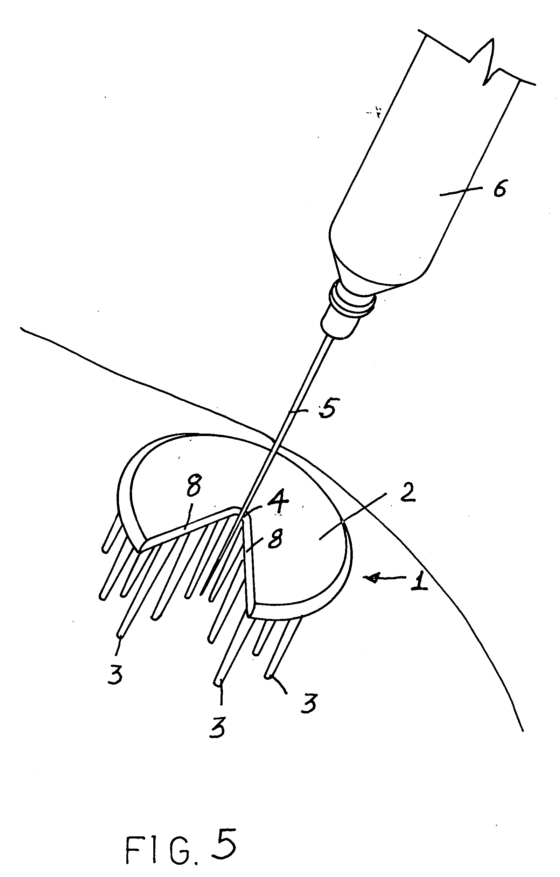

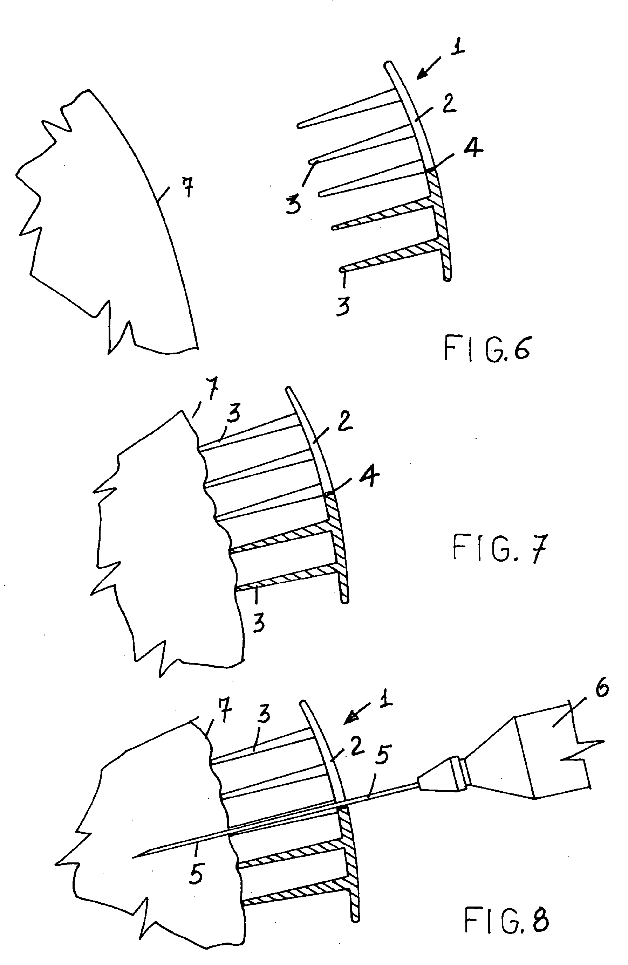

With reference to figures 5, 6, 7 and 8, they show a modified embodiment of the auxiliary device 1, which, in this embodiment, comprises a notch or incision 4, formed at the plate-like portion 2.

This notch or incision 4, in the exemplary embodiments shown in figures 5, 6, 7 and 8, has a V-shape and is formed or provided in the plate-like portion 2 of the auxiliary device 1, thereby allowing the needle 5 to be perfectly arranged.

Thus, said needle 5 will be properly guided at the time in which the operator selects the point of the skin 7 for performing the injection.

In this connection it should be apparent that the above mentioned notch 4, delimited by the sides 8, could have any other suitable configuration, thereby allowing the needle 5 to be easily located inside said notch 4.

Thus, said notch 4 will provide a guide element for properly introducing the needle 5 into the patient skin 7.

Thus, the injection operation would be greatly facilitated, with a combined effect of attenuating the pain sensation, while simultaneously reducing and eliminating the fear con condition of the patient.

The auxiliary device can comprise a handle or other gripping element, formed as a single body with the plate-like portion, for example, for easily handling the auxiliary device during the use thereof.

The auxiliary device according to the present invention is used as follows.

Before performing an injection, the device 1 is pressed on the skin 7 of the patient by a suitable pressure, so as to allows the points 3 to sensitize the skin 7 before inserting the syringe needle 5 and performing the injection proper.

The pressure will be held during the injection up to the penetration of the needle into the skin and underlying tissues.

Thus, the above mentioned points 3 applied on the user skin will "mask" the puncture proper performed by the needle 5.

In this connection it should be pointed out that the pressure to be applied to the auxiliary device and the duration, before and during the injection, will be set by the operator according to the contingent cases.

It has been found that the invention fully achieves the intended aim and objects.

In fact, the invention provides a device which has been specifically designed for reducing pain associated with injections of needles of any nature, shape and/or use, both in the human and veterinary fields: intradermal injection needles, subcutaneous injection needles, intramuscular injection needles, intravenous injection needles, intra-arterial injection needles, electromyography needles and the like.

It should be moreover apparent that the shape, number, size, arrangement, materials, density, stiffness of the points can be changed depending on requirements and the application as well as the type of used injection needle.

Advantageously, the device can also comprise a support element for supporting the injection syringe, so as to properly drive and preset the injection point.

Further advantageously, the device can be packaged in sterile packagings.

Moreover, the auxiliary device according to the present invention can be advantageously used both in a human and in a veterinary field.

In practicing the invention, the used materials, as well as the contingent size and shapes, can be any depending on the specific requirements.

## Claims

1. An auxiliary device for attenuating pain in performing injections, **characterized in that** said auxiliary device comprise a plate portion including a plurality of pointed elements adapted to be pressed on a user skin at a region about or near an injection point, to reduce a needle puncture pain.

2. A device, according to claim 1, **characterized in that** said device comprises one or more holes for allowing a syringe needle to be passed therethrough for performing an injection.

3. A device, according to claim 1, **characterized in that** said plate portion and said pointed elements are made as a single body.

4. A device, according to claim 1, **characterized in that** said plate portion comprises a disc including a plurality of points forming said pointed elements.

5. A device, according to claim 1, **characterized in that** said device is made of an injection molding plastic material.

6. A device, according to claim 1, **characterized in that** said device comprises a handle element formed as a single piece with said plate portion and adapted to allow said device to be easily handled in an use condition thereof

7. A device, according to claim 1, **characterized in that** said device can be pressed on a patient skin by such a pressure as to allow said pointed elements to sensitize said skin before and during an insertion of said syringe needle to perform an injection proper.

8. A device, according to claim 1, **characterized in that** said device is adapted to be used both in a human and in a veterinary field in association with intradermal injection, subcutaneous, intramuscular, intravenous, intra-arterial, electromyography needles and so on.

9. A device, according to claim 1, **characterized in that** said device comprises moreover a support element for mounting an injection syringe thereon, so as to guide and precisely preset the injection point.

10. A device, according to claim 1, **characterized in that** said device is packaged in a sterile packaging.

11. A device, according to claim 1, **characterized in that** said device further comprises a notch formed in the plate-like portion of said device.

12. A device, according to claim 11, **characterized in that** said notch has a substantially V-shape.

13. A device according to claim 1, **characterized in that** said device is adapted to be used for reducing pain caused by injection operations, traumas, small lesions, hair removing operations and inflammation conditions.
